(19) Europäisches Patentamt
European Patent Office
Office européen des brevets

(11) Veröffentlichungsnummer: **0 237 916 A2**

# (12) EUROPÄISCHE PATENTANMELDUNG

(21) Anmeldenummer: **87103390.8**

(22) Anmeldetag: **10.03.87**

(51) Int. Cl.⁴: **C07D 249/08**, C07D 233/56, A61K 31/41, A61K 31/45, A61K 31/00

(30) Priorität: **15.03.86 DE 3608727**

(43) Veröffentlichungstag der Anmeldung: **23.09.87 Patentblatt 87/39**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Ehrhardt, Heinz, Dr.**
**Bergstrasse 21**
**D-8901 Rehling(DE)**
Erfinder: **Blume, Ernst, Dr.**
**Rossertstrasse 20**
**D-6239 Kriftel(DE)**
Erfinder: **Raether, Wolfgang, Dr.**
**Falkensteinstrasse 6**
**D-6072 Dreieich(DE)**
Erfinder: **Dittmar, Walter, Dr.**
**Uhlandstrasse 10**
**D-6238 Hofheim am Taunus(DE)**

(54) **1, 1-Disubstituierte Cyclopropanderivate, Verfahren zu ihrer Herstellung sowie ihre Verwendung als Antimykotika oder als Zwischenprodukte.**

(57) Verbindungen I

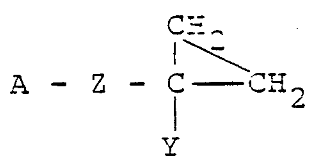

mit
A gleich t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazoyl, Benzofuryl, Benzothienyl,
Z gleich CO, $CH_2$ bzw. Resten von Ketonabkömmlingen;
Y gleich Azol oder gleich $-X-R^8$ ($R^8$ = (Cyclo-)Alkyl, Aromaten) oder gleich einer Anzahl von Amin-Abkömmlingen oder gleich Acyl
sind Antimykotika. Herstellung und Verwendung als Arzneimittel werden beschrieben.

EP 0 237 916 A2

## 1,1-Disubstituierte Cyclopropanderivate, Verfahren zu ihrer Herstellung sowie ihre Verwendung als Antimykotika oder als Zwischenprodukte

Die Erfindung betrifft 1,1-disubstituierte Cyclopropanderivate, mit antimykotischen Eigenschaften, sowie Verfahren zu ihrer Herstellung. Die Verbindungen sind ebenfalls wertvolle Zwischenprodukte für die Herstellung anderer biologisch aktiver Stoffe.

1-Methyl-bzw. 1-Phenylthio-1-benzoylcyclopropan sind aus CA87 , 134 289 ff bekannt ohne Angabe von Verwendung oder biologischer Wirkung.

Aus der EP-A2 88 050 sind photohärtbare gefärbte Massen bekannt, bestehend aus a) einem olefinisch ungesättigten, photopolymerisierbaren Bindemittel, b) einem Pigment oder einem Farbstoff und c) einem Photoinitiator der Formel

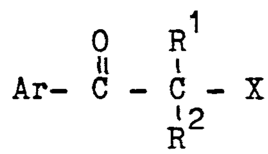

in welcher Ar Thio-bzw. Sulfinyl-aryl-und $R^1$ und $R^2$ neben einer Vielzahl anderer Bedeutungen auch $(C_2\text{-}C_8)$-Alkylen bedeuten können, während X eine (substituierte) Aminogruppe ist. Es finden sich jedoch weder irgendwelche Angaben über solche Cycloalkyl-Verbindungen im Text, noch experimentelle Angaben über die Herstellung solcher Verbindungen in den Beispielen. Andere Anwendbarkeiten als ihre Eignung als Photoinitiatoren sind auch nicht genannt worden.

Aus der EP-A 164 246 sind antifungal wirkende Verbindungen der Formel

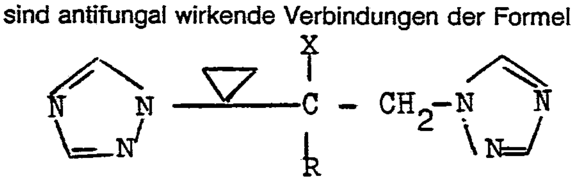

mit X = OH (verestert oder verethert), F, Cl oder Br und R = Phenyl (unsubstituiert oder substituiert) oder 5-Chlorpyrid-2-yl bekannt.

Diese bekannten Verbindungen lassen jedoch in ihrer Wirksamkeit, besonders in ihrer antimykotischen Wirksamkeit noch manchen Wunsch offen.

Der vorliegenden Erfindung lag die Aufgabe zugrunde bestimmte 1,1-disubstituierte Cyclopropanderivate mit biologisch interessanter Wirkung herzustellen, sowie einfache Verfahren zu ihrer Herstellung zu erarbeiten. Überraschenderweise besitzen die Cyclopropanderivate der erfindungsgemäßen Struktur antimykotische Wirkung oder können als Zwischenprodukte zur Herstellung von pharmazeutischen Präparaten oder Stabilisatoren Verwendung finden.

Die Erfindung ist daher auf Verbindungen der Formel (I) gerichtet

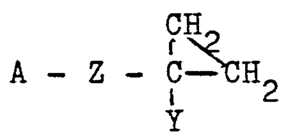 (I)

in welcher bedeuten:

A t-Butyl, Phenyl, Biphenyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-tetrahydrona phthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche

F, Cl, Br, J, $(C_1\text{-}C_8)$-Alkyl, geradkettig oder verzweigt und unsubstituiert oder mit 1-9 F-oder Cl-Atomen substituiert, $(C_3\text{-}C_8)$-Cycloalkyl, $(C_1\text{-}C_8)$-Alkoxy, geradkettig oder verzweigt, und unsubstituiert oder mit 1-9 F-oder Cl-Atomen substituiert, $(C_3\text{-}C_8)$Cycloalkyl-$(C_1\text{-}C_4)$Alkyl, $(C_1\text{-}C_8)$Alkylthio, $(C_1\text{-}C_8)$-Alkylsulfinyl-und sulfonyl, $NO_2$, CN sind;

Z $C=O$, $CH_2$, 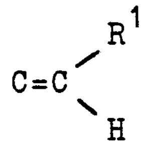 , 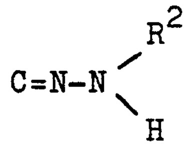 , $C=N-OR^3$ oder

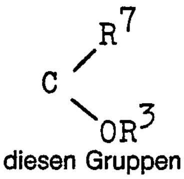

worin in diesen Gruppen Z

$R^1$ für Wasserstoff, $(C_1-C_{12})$Alkyl, Cyano, $(C_1-C_{10})$-Alkoxycarbonyl, $C_1-C_6$-Alkylcarbonyl, jeweils unsusbtituiertes oder mit F, Cl, Br ein-bis dreifach substituiertes Phenylcarbonyl oder Phenyl steht, wobei die Substituenten gleich oder verschieden sind

$R^2$ für Wasserstoff, $C_1-C_4$-Alkyl oder unsubstituiertes oder ein-bis dreifach mit F, Cl, Br substituiertes Phenyl steht, wobei die Substituenten gleich oder verschieden sind

$R^3$ Wasserstoff, $C_1-C_6$-Alkyl, $C_5-C_6$-Cycloalkyl, $(C_2-C_6)$-Alkyl, das mit 1-3 Chlor-oder Bormatomen substituiert ist, $C_2-C_6$-Alkenyl, unsubstituiert oder mit Chlor oder Brom ein-oder zweifachsubstituiert, $C_3-C_6$-Alkinyl, Geranyl, Farnesyl, $(C_1-C_4)$Alkoxycarbonyl-$(C_1-C_3)$-Alkyl, 1,2,4-Triazolylmethyl, Phenyl$(C_1-C_4)$-alkyl oder Phenoxy$(C_1-C_6)$-alkyl, Phenyl oder Pyridyl bedeutet und die 4 letztgenannten Gruppen jeweils unsubsti tuiert oder ein-bis dreifach F, Cl, Br, $CF_3$, $(C_1-C_4)$-Alkyl-, $(C_1-C_4)$-Alkoxy-, Phenoxy-, CN,$NO_2$, COOH-oder $(C_1-C_4)$-Alkoxycarbonyl substituiert sind, wobei die Substituenten gleich oder verschieden sind,

$R^3$ aber auch für Reste

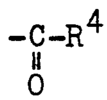 oder 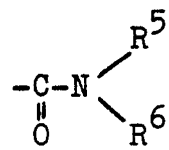

steht, in denen

$R^4$ $C_1-C_6$-Alkyl, $C_5$-oder $C_6$-Cycloalkyl, $C_2-C_6$-Alkenyl, Phenyl, Naphthyl oder Phenyl-$(C_1-C_4)$-alkyl bedeutet, wobei die drei letztgenannten Reste unsubstituiert oder ein-bis dreifach durch F, Cl, Br, $C_1-C_4$-Alkyl oder $C_1-C_4$-Alkoxy wobei die Substituenten gleich oder verschieden sind, oder einfach durch eine Trifluor-oder Trichlormethylgruppe substituiert sind und

$R^5$ und $R^6$ gleich oder verschieden sind und die Bedeutung von Wasserstoff, $C_1-C_6$-Alkyl, unsubstituiertes oder 1-3fach mit F,Cl,Br substituiertes Phenyl haben, jedoch nicht beide gleichzeitig H oder Phenyl sein dürfen,

$R^7$ für Wasserstoff, $C_1-C_6$-Alkyl, $C_2-C_3$-Alkinyl, Phenylethinyl, Benzyl oder Phenyl steht, wobei die Phenylreste jeweils unsusbtituiert oder durch 1 bis 2 F, Cl, Br, $C_1-C_4$-Alkyl-oder $C_1-C_4$-Alkoxygruppen substituiert sind, wobei die Substituenten gleich oder verschieden sind,

Y a)

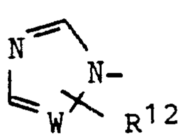

mit W = CH oder N

und $R^{12}$ gleich $(C_1-C_4)$-Alkyl oder $CF_3$

b) -X -$R^8$

mit X = -O-, -S-, S = O $SO_2$ und

$R^8$ = $(C_1-C_{12})$-Alkyl, geradkettig oder verzweigt $(C_3-C_8)$-Cycloalkyl, $(C_3-C_8)$Cycloalkyl-$(C_1-C_4)$-alkyl, Phenyl, Biphenyl, Phenoxyphenyl, Phenylthiophenyl, Phenyl$(C_1-C_4)$-alkyl, Naphthyl, Biphenyl$(C_1-C_4)$-alkyl, Phenylthiophenyl$(C_1-C_4)$-alkyl, Phenoxyphenyl-$(C_1-C_4)$Alkyl, Naphthyl$(C_1-C_4)$-alkyl, Benzthiazol-2-yl, Benzimidazol-2-yl, N-$(C_1-C_4)$-alkylbenzimidazol-2-yl, Pyridyl, Pyrimidin-2-yl, Furfuryl, Thienyl-2-methyl, wobei die genannten Ringsysteme unsubstituiert oder durch 1, 2 oder 3 Substituenten, die gleich oder verschieden sind und jeweils F, Cl, Br, J, $CF_3$ $(C_1-C_4)$-Alkyl, $(C_1-C_4)$-Alkoxy bedeuten, substituiert sind,

c)

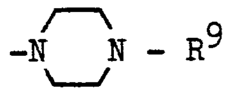

mit $R^9$ = $(C_1$-$C_8)$-Alkyl, $(C_5$-$C_8)$-Cycloalkyl, Acetyl, Phenyl, Benzyl; wobei der Phenylkern jeweils unsubstituiert oder mit 1-3 F, Cl, Br, J, $CF_3$, $(C_1$-$C_4)$-Alkyl-oder $(C_1$-$C_4)$-Alkoxygruppen substituiert ist, die gleich oder verschieden sind,

d)

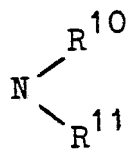

mit $R^{10}$ und $R^{11}$ gleich oder verschieden $(C_1$-$C_4)$Alkyl

e)

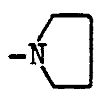
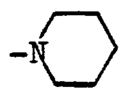
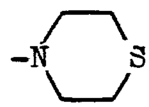
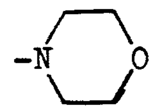

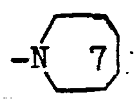
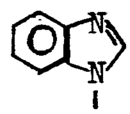
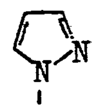
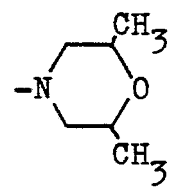

f) $-O\overset{O}{\overset{\parallel}{C}}R^{13}$ mit $R^{13}$ gleich $(C_1$-$C_8)$-Alkiyl, $(C_3$-$C_6)$-Cycloalkyl, Phenyl, Benzyl, Phenoxyphenyl, wobei die Phenylkerne jeweils unsubstituiert oder mit 1-3 F, Cl, Br, J, $CF_3$, $NO_2$, $(C_1$-$C_4)$-Alkyl oder $(C_1$-$C_4)$-Alkoxygruppen substituiert sind, die gleich oder verschieden sind, und ihre Salze mit physiologisch verträglichen Säuren, mit Ausnahme der Verbindungen, in denen

a) gleichzeitig Z = C=O, A = Phenyl und Y = S-$CH_3$ oder S-Phenyl sind; oder

b) gleichzeitig Z = C=O,

A = Phenyl, gegebenenfalls substituiert mit 1-3 Substituenten (die unabhängig voneinander ausgewählt sind aus F,Cl,Br,J,$CF_3$, $(C_1$-$C_4)$-Alkyl und $(C_1$-$C_4)$-Alkoxy) oder 5-Chlorpyrid-2-yl und Y = 1,2,4-Triazol-1-yl sind.

Die Erfindung betrifft auch Salze oder Quaternisierungsprodukte der Verbindung der Formel I, die durch Anwesenheit eines N-Atoms befähigt sind, solche Derivate zu bilden.

Bevorzugt unter den Verbindungen der Formel I sind solche worin mindestens einer der Substituenten folgende Bedeutung hat:

A: Phenyl, Biphenyl, 1,2,3,4-Tetrahydronaphthyl, Thienyl, Indanyl, jeweils unsubstituiert oder im aromatischen Ring mit ein oder zwei Substituenten, die gleich oder verschieden sind und jeweils F, Cl, Br, $CF_3$, $(C_1$-$C_4)$-Alkyl oder $(C_1$-$C_4)$-Alkoxy bedeuten, substituiert sind

Y: a)

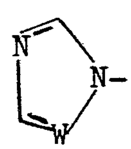

mit W = CH oder N

b) -X-$R^8$ mit

X = O, S

$R^8$ = $(C_1$-$C_{12})$-Alkyl, geradkettig oder verzweigt, Phenyl, Biphenyl, Phenoxyphenyl, Phenylthiophenyl, Phenyl-$(C_1$-$C_2)$Alkyl, Naphthyl, Biphenyl$(C_1$-$C_2)$alkyl, Naphthyl$(C_1$-$C_2)$alkyl, Benzthiazol-2-yl, Benzimidazol-2-yl, Furfuryl, Thienyl-2-methyl

wobei die genannten Ringsysteme unsubstituiert oder durch 1, 2 oder 3 Substituenten, die gleich oder verschieden sind und jeweils F, Cl, Br, $CF_3$, Methyl, Methoxy-Gruppen bedeuten, substituiert sind

c)

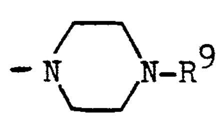

mit

$R^9$ = Phenyl, Benzyl, jeweils unsusbtituiert oder mit 1 oder 2 F, Cl, Br, $CF_3$, Methyl oder Methoxy-Gruppen substituiert,

Z:

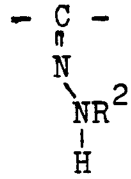 , 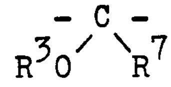

oder 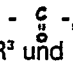, wobei $R^2$, $R^3$ und $R^7$ genannten Bedeutungen haben.

Ganz besonders bevorzugt sind Verbindungen, in denen mindestens einer der Substituenten folgende Bedeutung hat:

A Phenyl, Thienyl, jeweils unsubstituiert oder substituiert durch 1 oder 2 F-oder Cl-Atome, Methyl, Methoxy

Y a) 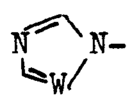

mit W = CH oder N

b) -X-$R^8$ mit X = O, S

$R^8$ = $(C_1$-$C_{12})$-Alkyl, geradkettig oder verzweigt, Phenyl, Benzyl, Naphthyl, Thienylmethyl, jeweils unsubstituiert oder 1 oder 2-fach substituiert durch F, Cl, Methyl, Methoxy

Z

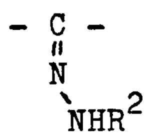 , 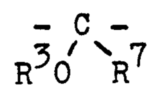

oder 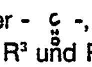, wobei $R^2$, $R^3$ und $R^7$ die genannten Bedeutungen haben und ihre Salze mit physiologisch verträglichen Säuren.

Die Cyclopropanderivate der Formel I sowie die Salze und Quaternisierungsprodukte können bei Wahl bestimmter Substituenten Chiralitätszentren aufweisen. Die Erfindung erfaßt daher sowohl Stereoisomerengemische als auch stereochemisch einheitliche Verbindungen der Formel I.

Die erfindungsgemäßen Cyclopropanderivate der Formel I sind, falls sie basische Verbindungen darstellen, zur Bildung von Salzen oder Quaternisierungsprodukten befähigt. Genannt seien Salze von organischen und anorganischen Säuren, wie beispielsweise Acetate, Fumarate, Oxalate, Benzoate, Phenolate, Nitrate, Bromide, Chloride, Sulfate, Sulfonate und Quaternisierungsprodukte mit Alkyl-und gegebenenfalls substituierten Phenylacylhaolgeniden.

Die Erfindung betrifft auch Verfahren zur Herstellung der Verbindungen der Formel I sowie ihrer Salze und Quaternisierungsprodukte, dadurch gekennzeichnet, daß man entweder

1) ein ω-Chlor-halogenketon der allgemeinen Formel II

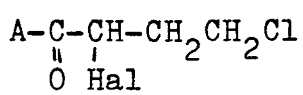 (II)

in welcher A die genannte Bedeutung und Hal für Chlor oder vorzugsweise Brom steht mit einem Nukleophil der Formel III

HY III,

in der Y die genannten Bedeutungen hat in Gegenwart eines Säureakzeptors und gegebenenfalls eines Verdünnungsmittels umsetzt oder

2) für den Fall, daß

Y = 1,2,4-Triazol-1-yl oder Imidazol-1-yl ist, ein Azolylketon der Formel IV

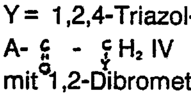 IV

mit 1,2-Dibromethan gegebenenfalls in Anwesenheit eines Verdünnungsmittels und eines Phasentransferkatalysators umsetzt und die erhaltenen Verbindungen der Formel I mit Z gleich C=O gewünschtenfalls nach laborüblichen Methoden weiter umwandelt sowie gewünschtenfalls in ihre Salze oder Quaternisierungsprodukte überführt,

Nach Verfahren 1 werden die Komponenten (II) und (III) zweckmäßigerweise etwa äquimolar eingesetzt. Es kann jedoch auch ein Unterschuß der einen oder anderen Komponente verwendet werden. Man arbeitet mit Temperaturen zwischen 0 und 120°C, vorzugsweise bei 10 bis 90°C.

Es wird dabei so vorgegangen, daß man entweder

a) in einem aprotischen dipolaren Lösungsmittel, bevorzugt Acetonitril oder Dimethylformamid, bei Anwesenheit von mindestens 2 Mol einer Base, vorzugsweise Kaliumcarbonat einstufig arbeitet, oder

b) in Aceton mit mindestens 1 Mol Base, vorzugsweise Kaliumcarbonat, zunächst ein Derivat der Formel V

$A - \underset{\overset{\|}{O}}{C} - \underset{Y}{CH} - CH_2CH_2Cl$ (V)

in welcher A und Y die oben angegebenen Bedeutungen haben, herstellt, gegebenenfalls isoliert und reinigt und dieses in Gegenwart einer wässerigen Lösung einer starken, anorganischen Base, der gegebenenfalls ein inertes Lösungsmittel, wie z.B. Toluol, Dichlormethan, Xylol etc. zugesetzt wird, und die einen Phasentransferkatalysator enthält, umsetzt. Unter einer starken Base wird in diesem Zusammenhang vorzugsweise konzentrierte, wässerige Natronlauge oder Kalilauge, bevorzugt eine mindestens 2normale Lauge, verstanden.

Geeignete Phasentransferkatalysatoren sind quaternäre Ammonium-oder Phosphoniumsalze sowie Kronether oder Polyethylenglykole. Solche Salze sind literaturbekannt. Als besonders geeignet für die vorliegenden Zwecke erwiesen sich Tetrabutylammoniumbromid und Benzyltriethylammoniumchlorid.

Nach Verfahren 2 werden die Komponenten IV und 1,2-Dibromethan zweckmäßigerweise etwa äquimolar eingesetzt. Es kann jedoch auch ein Unterschuß der einen oder anderen Komponente verwendet werden. Man arbeitet mit Temperaturen zwischen 30 und 150°C, vorzugsweise bei 50 bis 100°C, in Gegenwart eines Säureakzeptors in mindestens molarer Menge. Es wird dabei so vorgegangen, daß man in Gegenwart einer wässerigen Lösung einer starken, anorganischen Base, der gegebenenfalls ein inertes Lösungsmittel, wie z.B. Toluol, 1,2-Dichlorethan, Xylol zugesetzt wird, und die einen Phasentransferkatalysator enthält, umsetzt. Unter einer starken Base wird in diesem Zusammenhang vorzugsweise konzentrierte, wässerige Natronlauge oder Kalilauge, bevorzugt eine mindestens 2normale Lauge, verstanden. Geeignete Phasentransferkatalysatoren sind die bei Verfahren 1b angegebenen.

Die als Ausgangssubstanzen dienenden ω-Chlorhalogenketone II können durch Halogenierung der entsprechenden ω-Chlorketone erhalten werden. Als Beispiele seien genannt: Phenyl-, 4-Chlorphenyl-, 4-Phenyl-phenyl-, 3-Nitro-phenyl-, 1-Naphthyl-, 4-Chlor-1-naphthyl-, 1,2,3,4-tetrahydro-6-naphthyl-, 2,5-Dimethyl-3-methoxycarbonyl-4-furyl-, 2-Phenyl-5-furyl-, 3-Chlor-thien-2-yl-, 5-Chlor-thien-2-yl-, 2,5-Dimethyl-isoxazol-3-yl-, 2-Ethyl-benzofuran-3-yl-, 2-Chlorbenzofuran-3-yl-, Benzo-thiophen-2-yl-, Benzothiophenyl-3-yl-, 6-Chlor-2-methoxycarbonyl-benzothiophen-3-yl-, 6-Chlor-3-methyl-benzothiophen-2-yl-, Isobenzofuran-1-yl-, Indolizin-1-yl-, 1-Methyl-indol-3-yl-1-brom-3-chlor-propyl-keton.

Die Nukleophile der Formel III sind allgemein bekannte Verbindungen der organischen Chemie. Als Beispiele seien genannt: 1,2,4-Triazol, Imidazol, Pyrazol, Benzimidazol, 4-Chlorthiophenol, 3,4-Dichlorphenol, Benzoesäure, 5-(2-Chlor-4-trifluormethyl-phenoxy)-2-nitro-benzoesäure, Piperidin, 1-Phenyl-piperazin, 2,6-Dimethylmorpholin.

Der Reaktionsverlauf sei beispielhaft an der Umsetzung des 1-Brom-3-chlor-propyl-4-phenyl-phenyl-ketons mit 1,2,4-Triazol aufgezeigt:

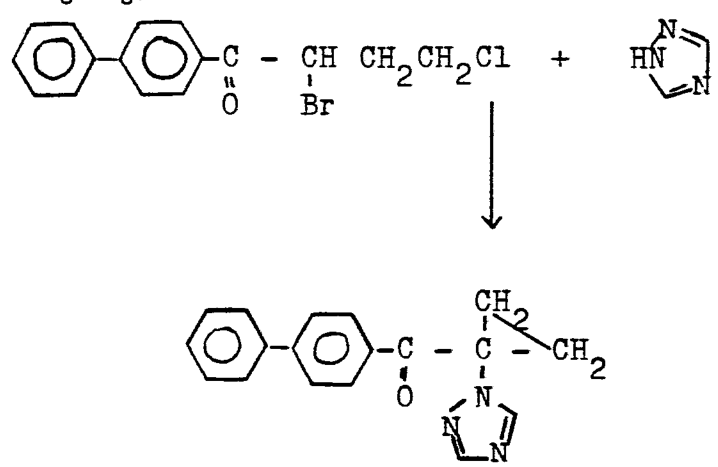

Der glatte Reaktionsverlauf ist überraschend, denn man mußte mit der Bildung der Monosubstitutionsprodukte sowie der Quaternisierungsprodukte rechnen.

Die Ketone können gewünschtenfalls nach laborüblichen Methoden in weitere erfindungsgemäßen Verbindungen überführt und diese gewünschtenfalls in Salze oder Quaternisierungsprodukte nach bekannten Methoden umgewandelt werden.

Die Verbindungen der Formel I und ihre Säureadditionssalze sind wertvolle Arzneimittel oder können als Zwischenpro dukte Verwendung finden. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitrol sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören phamazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen vorzugsweise in einer Konzentration von etwa 0,1 bis 99,0, vorzugsweise von etwa 0,5 bis 90 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human-und Veterinärmedizin zur Verhütung, Besserung und/oder Heilung der oben angeführten Erkankungen. Die Wirkstoffe oder die pharmazeutischen Zubereitungen können lokal, oral, parenteral, intraperitoneal und/oder rectal appliziert werden.

Im allgemeinen hat es sich sowohl in der Human-als auch in der Veterinärmedizin als vorteilhaft erwiesen, den oder die erfindungsgemäßen Wirkstoffe in Gesamtmengen von mindestens etwa 0,05, vorzugsweise 0,1, insbesondere 0,5 mg und von höchstens 200, vorzugsweise 100, insbesondere 30 mg/kg Körpergewicht je 24 Stunden, bezogen auf einen Erwachsenen des Gewichts von 75 kg, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arnzeimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel 1 -Verfahrensvariante 1a)

4-Phenyl-phenyl-[1-(1,2,4-triazol-1-yl)-cyclopropyl]-keton

Man suspendierte 44,7 g (0,648 mol) 1,2,4-Triazol und 89,5 g (0,648 mol) wasserfreies Kaliumcarbonat in 525 ml Acetonitril und gab portionsweise bei Rückfluß 202 g (0,6 mol) 1-Brom-3-chlor-propyl-4-phenyl-phenyl-keton in 20 Minuten zu. Nach 3 Stunden Reaktionszeit bei Rückfluß goß man auf Eiswasser und saugte die erhaltenen Kristalle ab. Man erhiet 162,0 g (93 % d.Theorie) farblose Kristalle vom Schmp. 154-5°C>

Beispiel 2 -Verfahrensvariante 1b)

4-Chlorphenyl-1-[4-(2-methoxyphenyl)-piperazinyl]cyclopropyl -keton

Man suspendierte 26,9 g (0,195 mol) wasserfreies Kaliumcarbonat in 70 ml Aceton, gab 30 g (0,156 mol) 1-(2-Methoxyphenyl)-piperazin zu und tropfte unter Kühlung bei 0° die Lösung von 38,5 g (0,13 mol) 1-Brom-3-chlor-propyl-4-chlorphenyl-keton in 50 ml Aceton gelöst zu. Man ließ über Nacht bei Raumtemperatur stehen, saugte die Salze ab und entfernte das Aceton i. Vak. Man nahm in Dichlormethan auf, - schüttelte mit Wasser aus, trocknete und entfernte das Solvens. Das verbleibende Öl (52,9 g) wurde in Ethylacetat aufgenommen und mit Chlorwasserstoff das Dihydrochlorid gefällt.

Man erhielt 38,5 g (62 % d.Th.) 3-Chlor-1-[4-(2-methoxyphenyl-piperazinyl]-propyl-4-chlorphenyl-keton-dihydrochlorid vom Schmp. 176°C (Ethanol).

Das so erhaltene Salz wurde in 200 ml Dichlormethan suspendiert, mit 100 ml 2 N Natronlauge versetzt, die organische Phase abgetrennt, mit 29 ml 50 %iger Natronlauge und 2,2, g Benzyltriethylammoniumchlorid versetzt und 5 h intensiv bei 20°C gerührt. Man verdünnte mit Wasser, trennt die organische Phase ab, wusch sie mit Wasser, trocknete und entfernte das Solvens im Vakuum. Das verbleibende Öl kristallisierte mit Methanol. Ausbeute: 25,2 g (85 % d.Th.) der Titelverbindung vom Schmp. 103-104°C

Herstellung des Ausgangsproduktes 1-Brom-3-chlor-propyl-4-phenyl-phenyl-keton

Man legte 246 g (0,95 mol) 3-Chlorpropyl-4-phenyl-phenylketon in 380 ml Eisessig vor und tropfte bei 30°C 152 g (0,95 mol) Brom zu, wobei unter Auflösung und Entfärbung die Reaktion einsetzte. Nach 1 h fiel ein dicker Niederschlag aus. Zur vollständigen Umsetzung wurde noch 4 h bei Zimmertemperatur gerührt. Man saugte die entstandenen Kristalle ab, wusch mit Wasser, löste in Dichlormethan, schüttelte mit $NaHCO_3$-Lösung und Wasser aus, trocknete und erhielt 250 g (78 % d.Th.) der Titelsubstanz als farblose Kristalle vom Schmp. 85-88°C.

Beispiel 3 -Verfahrensvariante 2

1-Naphthyl-[1-(1,2,4-triazol-1-yl)-cyclopropyl]-keton

Man legte 20,7 g (0,1 mol) (1,2,4-Triazol-1-yl)-methyl-1-naphthyl-keton in 100 ml Dichlorethan, 0,3 g Benzyltriethylammoniumchlorid und 27 ml 50 %ige Natronlauge vor und tropfte in 15 min bei Rückfluß 20,7 g (0,11 mol) 1,2-Dibromethan zu. Man erhitzte weitere 5 h auf Rückfluß, kühlte ab, verdünnte mit Wasser und schüttelte mit Dichlorethan aus. Nach Trocknen und Entfernen des Solvens erhielt man 10,6 g (45 % d.Th.) der Titelverbindung als Öl.

Beispiel 4

4-Chlorphenyl-4-fluorphenyl-[1-(1,2,4-triazol-1-yl)-cyclopropyl]-carbinol

Man stellte aus 21,0 g (0.12 mol) 4-Fluorbrombenzol und 3,0 g (0,126 mol) Magnsiumspänen in 60 ml Ether das 4-Fluorphenylmagnesiumbromid her und tropfte bei Zimmertemperatur die Lösung von 15 g (0,06 mol) 4-Chlorphenyl-[1-(1,2,4-triazol-1-yl)-cyclopropyl]-keton in 30 ml Tetra hydrofuran zu. Man erhitzte 2 h auf Rückfluß, zersetzte unter Kühlung mit gesättigter wäßriger Ammoniumchloridlösung und extrahierte mit Dichlormethan. Nach Abdestillieren des Solvens im Vakuum verblieben 15,0 g (73 % d.Th.) der Titelverbindung als farblose Kristalle vom Schmp. 183-184°C (aus Ethanol).

Beispiel 5

3-(4-Phenyl-phenyl)-3-[1-(1,2,4-triazol-1-yl)-cyclopropyl]-acrylsäureethylester

Man legte 17,4 g (0,06 mol) der Verbindung aus Beispiel 1 und 14,8 g (0,066 mol) Diethylphosphonylessigsäureethylester in 66 ml Dimethylformamid vor und gab in Portionen 1,98 g (0,066 mol) 80 % Natriumhydrid zu. Unter Gasentwicklung und Erwärmung setzte die Reaktion ein, die nach 5-stündigem Rühren bei 25°C beendet ist. Man goß auf Eiswasser und erhielt durch Absaugen der entstandenen Feststoffe 21,4 g (99 %) der Titelverbindung als bräunliche Kristalle vom Schmp. 98 -100°C. Zur Reinigung wurde über eine kurze Kieselgelsäule mit $CH_2Cl_2$ / Ethanol = 20:1 als Elutionsmittel chromatographiert, Schmp. 140 -142°C.

Beispiel 6

4-Chlorphenyl-[1-(1,2,4-triazol-1-yl)-cyclopropyl]-keton hydrazon

Man erhitzte 37,1 g (0,15 mol) 4-Chlorphenyl-[1-(1,2,4-triazol-1-yl)-cyclopropyl]-keton mit 37,5 g (0,3 mol) 80 proz. Hydrazinhydrat in 150 ml Ethanol 6 Stunden unter Rückfluß. Nach Abdestillieren des Lösungsmittels im Vakuum erhielt man 38,5 g (98 %) der Titelverbindung als gelbes Öl.

Beispiel 7

1-(4-Chlorbenzyl)-1-(1,2,4-triazol-1-yl)-cyclopropan

Man erhitzte die Suspension von 24,8 g (0,1 mol) 4-Chlorphenyl-[1-(1,2,4-triazol-1-yl)cyclopropyl]-keton mit 19,8 g 80 % Hydrazinhydrat in 40 ml Diethylenglykol 1 Stunde unter Rückfluß, kühlte ab und versetzte die klare gelbe Lösung mit 14,3 g gepulvertem Kaliumhydroxid. Man erhitzte unter Rückfluß, destillierte dann das entstandene Reaktionswasser ab bis wiederum Diethylenglykol refluxierte. Nach 1 Stunde wurde auf Wasser gegossen und mit Dichlormethan extrahiert; nach dem Trocknen wurde das Solvens im Vakuum entfernt und der verbleibende Rückstand in Hochvakuum destillierte. Man erhielt 13,5 g (57 %) der Titelverbindung als gelbliches Öl vom Kp 0,05 = 154 -156°C, das kristallisierte Schmp. 67-8°C.

Beispiel 8

4-Chlorphenyl-[1-(1,2,4-triazol-1-yl)-cyclopropyl]-keton-oxim

Man erhitzte 24,8 g (0,1 mol) 4-Chlorphenyl-[1,2,4-triazol-1-yl)-cyclopropyl]-keton mit 10,4 g Hydroxylaminhydrochlorid, 10,6 g Natriumacetat in 200 ml Ethanol und 100 ml Wasser 4 Stunden unter Rückfluß, entfernte die Lösungsmittel im Vakuum und verrieb den erhaltenen Kristallbrei mit Wasser. Man erhielt 21,7 g (83 %) der Titelverbindung als farblose Kristalle vom Schmp. 186 -218°C.

Beispiel 9

4-Chlorphenyl-[1-(1,2,4-triazol-1-yl)-cyclopropyl]-keton-oxim-methylcarbamat

Man legte 10 g (0,038 mol) der in Beispiel 8 erhaltenen Verbindung in 30 ml Aceton vor, gab 2,9 g Methylisocyanat und 2 Tropfen Triethylamin zu. ·Nach 3 Stunden Rückfluß saugte man den suspendierten Feststoff ab und isolierte 2,9 g Ausgangsprodukt. Aus der Mutterlauge wurden durch Abdestillieren im Vakuum 8,5 g (98 %) der Titelverbindung als farblose Kristalle von Schmp. 116 -118°C isoliert.

Beispiel 10

4-Fluorphenyl-[1-(1,2,4-triazol-1-yl)-cyclopropyl]-carbinol

Man versetzte die Lösung von 13,8 g (0,06 mol) 4-Fluorphenyl-[1-(1,2,4-triazol-1-yl)-cyclopropyl]-keton in 50 ml Methanol mit 1,2 g Natriumborhydrid, erhitzte 1 Stunde unter Rückfluß und isolierte 12,4 g (89 %) der Titelverbindung durch Aufarbeiten mit Dichlormethan/Wasser, Schmp. 104 -106°C.

Beispiel 11

4-Chlorphenyl-[1-(1,2,4-triazol-1-yl)-cyclopropyl]-methyl-4-trifluormethylbenzylether

Man legte 16,3 g (0.065 mol) der nach Beispiel 61 erhaltenen Verbindung in 50 ml Dimethylformamid vor, gab 2,16 g 80 proz. Natriumhydrid zu, rührte bis die Gasentwicklung beendet ist (ca. 1 Stunde) und tropfte 14,0 g 4-Trifluormethylbenzylchlorid zu, wobei die Innentemperatur von 30°C auf 67°C stieg. Man erhitzte 4 Stunden auf 80°C und arbeitete mit Dichlormethan/Wasser auf. Zur Reinigung wurde destilliert; man erhielt 23,1 g (87 %) der Titelverbindung als gelbliches Öl vom Kp 0,03 = 195 -197°C.

Gemäß den Beispielen 1 bis 11, jedoch unter Verwendung entsprechender Verbindungen der Formeln II, III und I mit $Z = -\overset{O}{\overset{\|}{C}}-$, wurden die nachfolgenden Verbindungen der Formel I hergestellt.

| Beispiel Nr. | A | Z | Y | Sdp.(Druck) bzw. Smp (°C) | Verf.-variante |
|---|---|---|---|---|---|
| 12 | 1-naphthyl | CO | 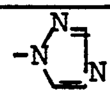 $\cdot HNO_3$ | 125 | 2 |
| 13 | $3,4-(CH_2)_4-C_6H_3$ | " | " | 182-184 (0,04) | 1a |
| 14 | $C_6H_5$ | " | 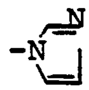 | 63-66 | 1b |
| 15 | $4-CH_3O-C_6H_4$ | " | " | Öl | 1b |
| 16 | $4-F-C_6H_4$ | " | " | 64-66 | 1b |
| 17 | $4-Cl-C_6H_4$ | " | " | 69-70 | 1b |
| 18 | $2,4-Cl_2-C_6H_3-$ | " | " $\cdot HNO_3$ | 158-160 | |
| 19 | $4-CH_3-C_6H_4$ | " | " | 88-90 | 1b |
| 20 | 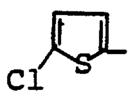 | " | 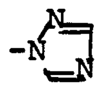 | 74-76 | 1a |
| 21 | 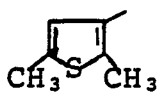 | " | " | 94-95 | 1a |
| 22 | " | " | 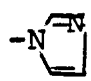 | 77-79 | 1a |
| 23 | 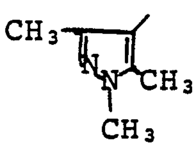 | " | 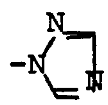 | 152-4 0,06 | 1a |
| 24 | $4-Cl-C_6H_4$ | " | $S-C_4H_9$ | 128 (0,1) | 1b |
| 25 | " | " | $S-C_8H_{17}$ | 170 (0,1) | 1b |
| 26 | " | " | $SCH_2-$ 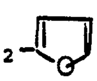 | 165-79 (0,1) | 1b |
| 27 | " | " | $SCH_2C_6H_3-2,4-Cl_2$ | 99-100 | 1b |
| 28 | " | " | $SCH_2C_6H_4-4-Cl$ | 74-75 | 1b |
| 29 | " | " | $S-C_6H_4-4-Cl$ | 104-105 | 1b |
| 30 | " | " | $S-C_6H_3-3,4-Cl_2$ | 65 | 1b |
| 31 | " | " | S-2-naphthyl | 82-84 | 1b |

| Beispiel Nr. | A | Z | Y | Sdp.(Druck) bzw. Smp (°C) | Verf.-variante nach Bsp. |
|---|---|---|---|---|---|
| 32 | 4-Cl-$C_6H_4$- | CO | 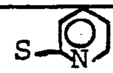 | 103-104 | 1b |
| 33 | " | " | 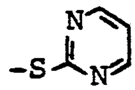 | 110-111 | 1b |
| 34 | " | " | $SO_2C_6H_5$ | 101-102 | 1b |
| 35 | " | " | $SO_2C_6H_4$-Cl | Öl | 1a |
| 36 | " | " | $SO_2CH_3$ | Öl | 1a |
| 37 | " | " | 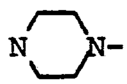-$C_6H_5$ | 132-133 | 1b |
| 38 | " | " | 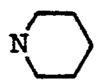 | Öl | 1b |
| 39 | " | " | $OC_6H_4$-4-Cl | 69-71 | 1b |
| 40 | " | " | $OC_6H_3$-2,4-$Cl_2$ | Öl | 1b |
| 41 | " | " | $OC_6H_3$-3,4-$Cl_2$ | Öl | 1b |
| 42 | " | " | $OC_6H_4OC_6H_3$-2,4-$Cl_2$ | Öl | 1b |
| 43 | 4-F-$C_6H_4$ | " | $SCH_2C_6H_4$-4-Cl | Öl | 1b |
| 44 | 4-$CH_3$-$C_6H_4$ | " | " | Öl | 1b |
| 45 | 4-$CH_3O$-$C_6H_4$ | " | " | Öl | 1b |
| 46 | 4-$C_6H_5$-$C_6H_4$ | " | 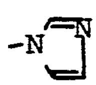 | 106-118 | 1a |
| 47 | 4-Cl-$C_6H_4$ | " | 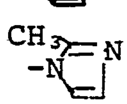 | 171-176 (0,1) | 1a |
| 48 | 4-$C_6H_5$-$C_6H_4$ | " | 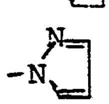 | 125-127 | 1a |
| 49 | 4-(4-F-$C_6H_4$O)$C_6H_4$ | | 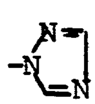 | 116-118 | 1a |
| 50 | 4-(4-Cl$C_6H_4$O)$C_6H_4$ | | " | 209-212 (0,04) | 1a |

| Bei-spiel Nr | A | Z | Y | Sdp (Druck) bzw. Smp (°C) | Verf. vari-ante Bsp. |
|---|---|---|---|---|---|
| 51 | $4-C_6H_5-C_6H_4$ | $-C-$<br>$HN-NH_2$ | " | 160-161 | 6 |
| 52 | 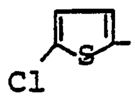 | $-C-$<br>$HN$<br>$NC_6H_5$<br>$H$ | " | 91-93 | 6 |
| 53 | " | $-C-$<br>$N-OCH_2-C_6H_4-4Cl$ | " | Öl | 8 |
| 54 | " | $-C-$<br>$N\ -OCH_2-C_6H_4-3-OC_6H_5$ | " | Öl | 8 |
| 55 | " | $-C-$<br>$N-OCH_2-C_6H_4-4-CF_3$ | " | Öl | 8 |
| 56 | $4-C_6H_5-C_6H_4$ | $-C-$<br>$N$<br>$OH$ | " | 195-198 | 8 |
| 57 | $4-Cl-C_6H_4-$ | $-CHOH-$ | 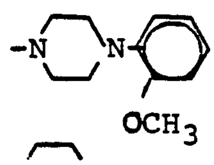 | 79-81 | 10 |
| 58 | " | " | 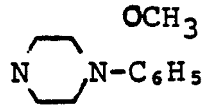 | 114-116 | 10 |
| 59 | " | " | 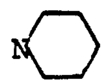 ·HCl | 231 | |
| 60 | $C_6H_5-$ | $-CH-$<br>$OH$ | 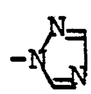 | 111-112 | 10 |
| 61 | $4-Cl-C_6H_4-$ | " | " | 150-151 | 10 |
| 62 | " | $-CH-$<br>$O-CH_2-C_6H_3-2,4-Cl_2$ | " | Öl | 11 |
| 63 | " | $-C-$<br>$CH_3\ \ OH$ | " | 153-5 | 4 |
| 64 | " | $-C-$<br>$C_2H_5\ \ OH$ | " | 143-145 | 4 |

| Bei-spiel Nr. | A | Z | Y | Sdp (Druck) bzw. Smp (°C) | Verf. vari-ante Bsp. |
|---|---|---|---|---|---|
| 65 | " | 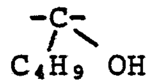 | " | 157-159 | 4 |
| 66 | " | 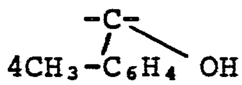 | " | 192-193 | 4 |
| 67 | " | 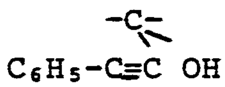 | " | 146-147 | 4 |
| 68 | 2,4-$Cl_2$-$C_6H_3$- | 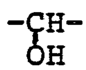 x $HNO_3$ | " | 144 (Z) | 10 |
| 69 | " | 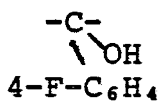 | " | 204-207 | 4 |
| 70 | " | 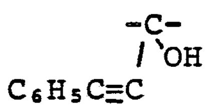 | " | 153-155 | 4 |
| 71 | 4-$C_6H_5$-$C_6H_4$- | 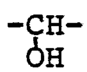 | " | 171-172 | 4 |
| 72 | 4-$C_6H_5$-$C_6H_4$- | 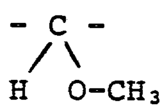 | 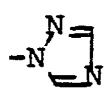 | 226/0,04 | 11 |
| 73 | " | 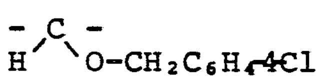 | " | 266-70/0,2 | 11 |
| 74 | 4-(4-$FC_6H_4O$)-$C_6H_4$- | 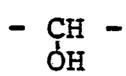 | " | 138-141 | 10 |
| 75 | " | 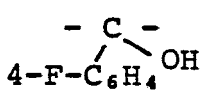 | " | 179-81 | 4 |
| 76 | 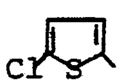 | " | " | 195-197 | 4 |

| Beispiel Nr. | A | Z | Y | Sdp (Druck) bzw. Smp (°C) | Verf. variante Bsp. |
|---|---|---|---|---|---|
| 77 | " | - C -<br>OH<br>4-Cl-$C_6H_4$ | " | 160-163 | 4 |
| 78 | " | - CH -<br>OH | " | 94-95 | 10 |
| 79 | 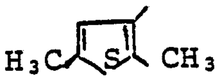 | - CH -<br>OH | " | 92-94 | 10 |
| 80 | " | - C -<br>OH<br>$C_6H_5C{\equiv}C$ | " $\cdot HNO_3$ | 94 (Z) | 4 |
| 81 | 4-Cl-$C_6H_4$ | -CH-<br>O-$CH_2$-$C_6H_4$-3-$CF_3$ | " | 209-11/0,05 | 11 |
| 82 | " | -CH-<br>O -$CH_2$-$C_6H_3$-2,6$Cl_2$ | " | 225-8/0,05 | 11 |
| 83 | " | -CH-<br>O$CH_2$- $C_6H_4$-3-O$C_6H_5$ | " | 241-5/0,02 | 11 |
| 84 | 3,4-$(CH_3)_2$-$C_6H_3$- | - CH -<br>O-$CH_2$-$C_6H_4$-4-$CF_3$ | " | 225-7/0,02 | 11 |
| 85 | " | - CH -<br>O-$CH_2$-$C_6H_4$-3-$CF_3$ | " | 221-4/0,05 | 11 |
| 86 | 4-Cl-$C_6H_4$- | -CH-<br>O- $CH_2$-$C_6H_4$-2-$CH_3$ | 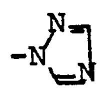 | 189-91/0,09 | 11 |
| 87 | 4-F-$C_6H_4$ | - CH -<br>O-$CH_2$-$C_6H_4$-4-$CF_3$ | " | 203-205/0,04 | 11 |
| 88 | 4-Cl-$C_6H_4$ | -CH-<br>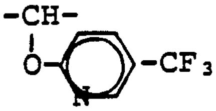 | " | 94-96 | 11 |
| 89 | " | -CH-<br>O-$C_6H_4$-4-$CF_3$ | " | 213-7/0,05 | 11 |
| 90 | " | -CH-<br>O-$C_6H_3$-2Cl-4-$CF_3$ | " | 224-6/0,03 | 11 |

| Bei- spiel Nr. | A | Z | Y | Sdp(Druck) bzw. Smp (°C) | Verf. vari- ante |
|---|---|---|---|---|---|
| 91 | " | 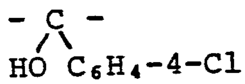 | " | 174-6 | 4 |
| 92 | $2,4-F_2-C_6H_3-$ | 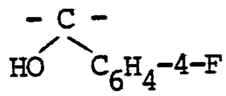 | " | 194-5 | 4 |
| 93 | $4-Cl-C_6H_4$ | 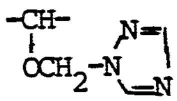 | " | 111-2 | 11 |
| 94 | $C_6H_5$ | CO | $-S-CH_2-C_6H_4-4-Cl$ | Öl | 1b |
| 95 | 1-naphthyl | " | " | | " |
| 96 | 2-naphthyl | " | " | | " |
| 97 | $3,4-(CH_2)_4-C_6H_3$ | " | " | | " |
| 98 | $3,4-(CH_2)_3-C_6H_3$ | " | " | | " |
| 99 | $4-C_6H_5-C_6H_4-$ | " | " | | " |
| 100 | $4-(4-F-C_6H_4O)C_6H_4$ | " | " | | " |
| 101 | 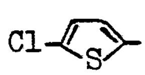 | " | " | Öl | " |
| 102 | " | " | $-S-CH_2-C_6H_5$ | | " |
| 103 | 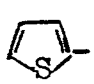 | " | 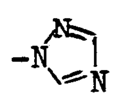 | Öl | 1a |
| 104 | $3,4-(CH_2)_3-C_6H_3$ | " | " | | " |
| 105 | $4-Cl-C_6H_4-$ | " | $S-CH_2-C_6H_5$ | Öl | 1b |

| Beispiel Nr. | A | Z | Y | Sdp(Druck) bzw. Smp (°C) | Verf. variante |
|---|---|---|---|---|---|
| 106 | 4-Cl-$C_6H_4$- | CO | $-S-CH_2-C_6H_4-4-F$ | | 1b |
| 107 | " | " | $-S-CH_2-C_6H_4-4-OCH_3$ | | " |
| 108 | " | " | $-S-(CH_2)_2-C_6H_4-4-Cl$ | | " |
| 109 | " | " | $-S-CH_2-$ 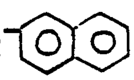 | | " |
| 110 | " | " | -O- 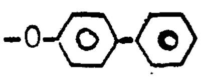 | 110-111 | " |
| 111 | 4-Cl-$C_6H_4$- | " | $-O-CH_2-C_6H_4-4-Cl$ | | " |
| 112 | " | " | $-O-CH_2-C_6H_3-2,4-Cl_2$ | | " |
| 113 | " | " | $-O-C_6H_4-4-CF_3$ | | " |
| 114 | " | " | 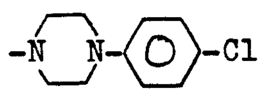 | | " |
| 115 | " | " | $-S-CH_2-C_6H_4-3-Cl$ | | " |
| 116 | " | " | $-S-CH_2-C_6H_4-2-Cl$ | | " |
| 117 | " | " | $-S-CH_2-C_6H_3-2,6-Cl_2$ | | " |
| 118 | 4-F-$C_6H_4$- | " | $-S-CH_2-C_6H_5$ | | " |
| 119 | " | " | $-S-CH_2-C_6H_4-4-Cl$ | Öl | " |
| 120 | " | " | $-S-CH_2-C_6H_3-2,4-Cl_2$ | | " |
| 121 | " | " | $-S-CH_2-C_6H_4-3-Cl$ | | " |

| Beispiel Nr. | A | Z | Y | Sdp(Druck) bzw. Smp (°C) | Verf. variante |
|---|---|---|---|---|---|
| 122 | 4-F-$C_6H_4$- | CO | -S-$CH_2$-$C_6H_4$-4-F | Öl | 1b |
| 123 | " | " | -S-$CH_2$-$C_6H_4$-4-$OCH_3$ | | " |
| 124 | " | " | -S-$CH_2$- 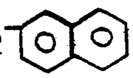 | | " |
| 125 | " | " | -S-$CH_2$-$C_6H_4$-2-Cl | | " |
| 126 | " | " | -S-$CH_2$-$C_6H_4$-3-F | | " |
| 127 | " | " | -S-$CH_2$-$C_6H_4$-2-F | | " |
| 128 | " | " | -O-$CH_2$-$C_6H_4$-4-Cl | | " |
| 129 | " | " | -O-$CH_2$-$C_6H_4$- 4-F | | " |
| 130 | 2,4-$CH_2$-$C_6H_3$- | " | -S-$(CH_2)_3$-$CH_3$ | 165-170 (0.1) | " |
| 131 | " | " | -S-$CH_2$-$C_6H_4$-4-Cl | | " |
| 132 | 2,4-$F_2$-$C_6H_3$- | " | -S-$CH_2$-$C_6H_5$ | | " |
| 133 | " | " | -S-$CH_2$-$C_6H_4$-4-Cl | Öl | " |
| 134 | " | " | 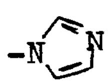 | | " |
| 135 | " | " | -S-$CH_2$-$C_6H_4$-4-F | Öl | " |
| 136 | " | " | -O-$CH_2$-$C_6H_4$-4-F | | " |
| 137 | " | " | -S-$CH_2$-$C_6H_3$-2,4-$Cl_2$ | | " |

| Beispiel Nr. | A | Z | Y | Sdp(Druck) bzw. Smp (°C) | Verf. variante |
|---|---|---|---|---|---|
| 138 | 2,4-F,Cl-$C_6H_3$- | CO | -S-$CH_2$-$C_6H_4$-4-Cl | 77-78 | 1b |
| 139 | 2,4-Cl,F-$C_6H_3$- | " | " | 62-63 | " |
| 140 | 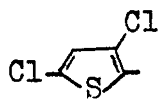 | " | -S-$CH_2$-$C_6H_4$-4-Cl | | " |
| 141 | " | " | 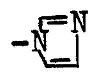 | | " |
| 142 | " | " | 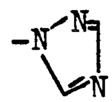 | | 1a |
| 143 | 4-Cl-$C_6H_4$- | -CH-<br>O-Geranyl | 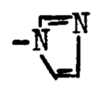 | Öl | 11 |
| 144 | " | -CH-<br>O-$CH_2$-$C_6H_4$-4-Cl | " | Öl | 11 |
| 145 | 4-Cl-$C_6H_4$- | -CH-<br>O-$CH_2$-$C_6H_3$-2,4-$Cl_2$ | " | 108 | 11 |
| 146 | " | -C-<br>$CH_2$ | " | 94-95 | 5[a)] |
| 147 | " | -C-<br>HC-$C_6H_4$-4-Cl | " | | 5[a)] |
| 148 | | -C-<br>HC-$C_6H_3$-2,4-$Cl_2$ | " | 116-117 | 5[a)] |

a) Die Herstellung erfolgte durch Umsetzung von 1-(4-Chlorbenzoyl)-1-(imidazol-1-yl)-cyclopropan mit den entsprechenden Triphenylphosphniumchloriden nach Beispiel 5, unter Verwendung von Dimethylsulfoxid als Lösungsmittel.

| Beispiel Nr. | A | Z | Y | Sdp(Druck) bzw. Smp (°C) | Verf. variante |
|---|---|---|---|---|---|
| 149 | t-butyl | CO | 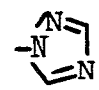 | 114-115 | 2 |
| 150 | 4-Cl-$C_6H_4$ | CO | 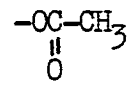 | 60-65 | 1b |
| 151 | 4-Cl-$C_6H_4$- | CO | 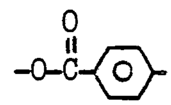 | 111-13 | 1b |
| 152 | " | " | 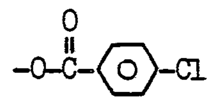 | | 1b |
| 153 | " | " | 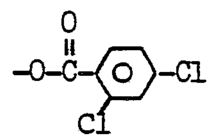 | | 1b |
| 154 | " | " | 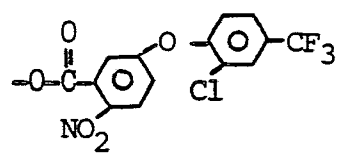 | | 1b |
| 155 | 2,4-$F_2$-$C_6H_3$ | CO | 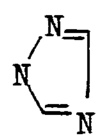 | Öl | 1a |
| 156 | " | -CHOH- | " | 127-9 | 10 |
| 157 | " | -CH-<br>\|<br>$OCH_2C_6H_4$-4-$CF_3$ | " | Öl | 11 |
| 158 | 4-$CH_3$-$C_6$-$H_4$ | CO | " | 112-3 | 1a |
| 159 | 4-$CH_3$-O-$C_6H_4$ | CO | " | 104-5 | 1a |
| 160 | 4-$C_6H_5$-$C_6H_4$ | CHOH | 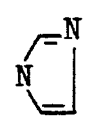 | 190-1 | 10 |

Als Beispiel für die hohe orale und parenterale in-vivo-Wirksamkeit der erfindungsgemäßen Verbindungen werden Behandlungsergebnisse an experimentell mit Candida albicans infizierten Labortieren angeführt.

Zur Feststellung der oralen und parenteralen Wirksamkeit wurden Gruppen von je 5 18-20 g schweren Mäusen (Stamm HOE: NMRKF; SPF 71) mit 2• $10^6$ Keimen/Tier infiziert.

Die Behandlung der Tiere erfolgte oral bzw. subcutan in 8 gleichen Einzeldosen zu je 30 mg/kg bzw. 10 mg/kg Körpergewicht (-24/-18/ -2h/ + 2/24/30/48/54h).

Neben der mit den erfindungsgemäßen Substanzen I behandelten Gruppe von 5 Tieren wurde zum Vergleich eine Gruppe von ebenfalls 5 Tieren mit der Referenzsubstanz Ketoconazol behandelt. Eine Kontrollgruppe von 10 Tieren blieb nach der Infektion unbehandelt.

Wie aus Tabelle 2 hervorgeht, fand sich bei den erfindungsgemäßen Verbindungen eine bis um das Doppelte verlängerte Überlebenszeit der Tiere nach der Infektion, verglichen mit dem derzeitigen Standardpräparat Ketoconazol.

Tabelle 2:

Ergebnisse Candida albicans NMRI-Maus (8malige Behandlung)

| Dosis | Beispiel-Nr. | Anzahl Tiere | Überlebenszeiten Tage nach Infektion | | | | | Tage $\bar{x}$ | Überlebenszeiten in % (Standardpräp. : 100 %) |
|---|---|---|---|---|---|---|---|---|---|
| oral 8 x 30 mg/kg | 91 | 5 | 8 | 8 | 10 | 10 | 10 | 9,2 | 139,3 |
| | 4 | 5 | 9 | 10 | 10 | 10 | 10 | 9,8 | 148,4 |
| | Ketoconazol | 5 | 6 | 6 | 7 | 7 | 7 | 6,6 | 100 |
| oral 8 x 10 mg/kg | 91 | 5 | 7 | 7 | 7 | 7 | 10 | 7,6 | 135,7 |
| | 4 | 5 | 8 | 10 | 10 | 10 | 10 | 9,6 | 171,4 |
| | Ketoconazol | 5 | 5 | 5 | 5 | 6 | 7 | 5,6 | 100 |
| subcutan 8 x 30 mg/kg | 91 | 5 | 8 | 8 | 8 | 9 | 10 | 8,6 | 113,1 |
| | 4 | 5 | 10 | 10 | 11 | 11 | 12 | 10,8 | 142,1 |
| | Ketoconazol | 5 | 7 | 7 | 7 | 8 | 9 | 7,6 | 100 |
| subcutan 8 x 10 mg/kg | 91 | 5 | 8 | 9 | 9 | 10 | 10 | 9,2 | 176,9 |
| | 4 | 5 | 8 | 11 | 12 | 13 | 14 | 11,6 | 223 |
| | Ketoconazol | 5 | 5 | 5 | 5 | 5 | 6 | 5,2 | 100 |
| Kontrollen infizierte Tiere unbehandelt | - | 10 | 1<br>2 | 1<br>2 | 1<br>2 | 1<br>2 | 2<br>3 | 1,7 | 27,2 |

## Ansprüche

1. Verbindung der Formel I

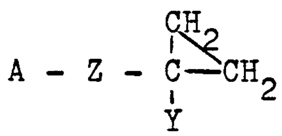

(I)

in welcher bedeuten:

A t-Butyl, Phenyl,Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche

F, Cl, Br, J, $(C_1-C_8)$-Alkyl, geradkettig oder verzweigt und unsubstituiert oder mit 1-9 F-oder Cl-Atomen substituiert, $(C_3-C_8)$-Cycloalkyl, $(C_1-C_8)$-Alkoxy, geradkettig oder verzweigt, und unsubstituiert oder mit 1-9 F-oder Cl-Atomen substituiert, $(C_3-C_8)$Cycloalkyl$(C_1-C_4)$Alkyl, $(C_1-C_8)$Alkylthio, $(C_1-C_8)$-Alkylsulfinyl-und sulfonyl, $NO_2$, CN sind;

Z C=O, $CH_2$, 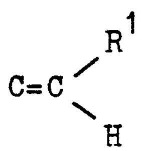 , 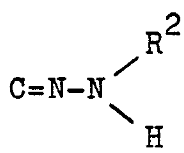 , $C=N-OR^3$ oder

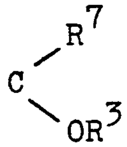

worin in diesen Gruppen Z

$R^1$ für Wasserstoff, $(C_1-C_{12})$Alkyl, Cyano, $(C_1-C_{10})$-Alkoxycarbonyl, $C_1-C_6$-Alkylcarbonyl, jeweils unsubstituiertes oder mit F, Cl, Br ein-bis dreifach substituiertes Phenylcarbonyl oder Phenyl steht, wobei die Substituenten gleich oder verschieden sind,

$R^2$ für Wasserstoff, $C_1-C_4$-Alkyl oder unsubstituiertes oder ein-bis dreifach mit F, Cl, Br substituiertes Phenyl steht, wobei die Substituenten gleich oder verschieden sind,

$R^3$ Wasserstoff, $C_1-C_6$-Alkyl, $C_5-C_6$-Cycloalkyl, $(C_2-C_6)$-Alkyl, das mit 1-3 Chlor-oder Bromatomen substituiert ist, $C_2-C_6$-Alkenyl, unsubstituiert oder mit Chlor oder Brom ein-oder zweifach substituiert, $C_3-C_6$-Alkinyl, Geranyl, Farnesyl, $(C_1-C_4)$Alkoxycarbonyl-$(C_1-C_3)$-Alkyl,1,2,4-Triazolylmethyl, Phenyl$(C_1-C_4)$-alkyl oder Phenoxy$(C_1-C_6)$-alkyl, Phenyl oder Pyridyl bedeutet und die 4 letztgenannten Gruppen jeweils unsubstituiert oder ein-bis dreifach durch F, Cl, Br, $CF_3$, $(C_1-C_4)$-Alkyl-, $(C_1-C_4)$-Alkoxy-, Phenoxy-, CN,$NO_2$, COOH oder $(C_1-C_4)$-Alkoxycarbonyl substituiert sind, wobei die Substituenten gleich oder verschieden sind,

$R^3$ aber auch für Reste - 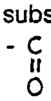 $-R^4$ oder

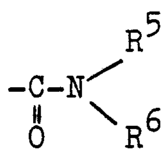

steht, in denen

$R^4$$C_1-C_6$-Alkyl, $C_5$-oder $C_6$-Cycloalkyl, $C_2-C_6$-Alkenyl, Phenyl, Naphthyl oder Phenyl-$(C_1-C_4)$-alkyl bedeutet, wobei die drei letztgenannten Reste unsubstituiert oder ein-bis dreifach durch F, Cl, Br, $C_1-C_4$-Alkyl oder $C_1-C_4$-Alkoxy, wobei die Substituenten gleich oder verschieden sind, oder einfach durch eine Trifluor-oder Trichlormethylgruppe substituiert sind und

$R^5$ und $R^6$ gleich oder verschieden sind und die Bedeutung von Wasserstoff, $C_1-C_6$-Alkyl, unsubstituiertes oder 1-3fach mit F,Cl,Br, substituiertes Phenyl haben, jedoch nicht beide gleichzeitig H oder Phenyl sein dürfen,

$R^7$ für Wasserstoff, $C_1-C_6$-Alkyl, $C_2-C_3$-Alkinyl, Phenylethinyl, Benzyl oder Phenyl steht,

wobei die Phenylreste jeweils unsubstituiert oder durch 1 bis 2 F, Cl, Br, $C_1-C_4$-Alkyl-oder $C_1-C_4$-Alkoxygrup-

pen substituiert sind, wobei die Substituenten gleich oder verschieden sind,
Y a)

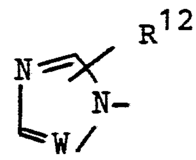

mit W = CH oder N
und $R^{12}$ gleich ($C_1$-$C_4$)-Alkyl oder $CF_3$ oder H
b) -X -$R^8$
mit X = O, S, S=O, $SO_2$ und
$R^8$ = ($C_1$-$C_{12}$)-Alkyl, geradkettig oder verzweigt, ($C_3$-$C_8$)-Cycloalkyl, ($C_3$-$C_8$)Cycloalkyl-($C_1$-$C_4$)-alkyl, Phenyl, Biphenylyl,Phenoxyphenyl, Phenylthiophenyl, Phenyl($C_1$-$C_4$)-alkyl, Naphthyl, Biphenyl ($C_1$-$C_4$)-alkyl, Phenylthiophenyl ($C_1$-$C_4$)-alkyl, Phenoxyphenyl-($C_1$-$C_4$) alkyl, Naphthyl($C_1$-$C_4$)-alkyl, Benzthiazol-2-yl, Benzimidazol-2-yl, N-($C_1$-$C_4$)-Alkylbenzimidazol-2-yl, Benzyl, Pyridyl, Pyrimidin-2-yl, Furfuryl, Thienyl-2-methyl,
wobei die genannten Ringsysteme unsubstituiert oder durch 1, 2 oder 3 Substituenten, die gleich oder verschieden sind und jeweils F, Cl, Br, J, $CF_3$, ($C_1$-$C_4$)-Alkyl, ($C_1$-$C_4$)-Alkoxy bedeuten, substituiert sind, c)

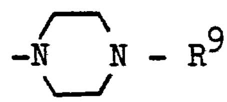

mit $R^9$ = ($C_1$-$C_8$)-Alkyl, ($C_5$-$C_8$)-Cycloalkyl, Acetyl, Phenyl, Benzyl, wobei der Phenylkern jeweils unsubstituiert oder mit 1-3 F, Cl, Br, J, $CF_3$, ($C_1$-$C_4$)-Alkyl-oder ($C_1$-$C_4$)-Alkoxygruppen substituiert ist, wobei die Substituenten gleich oder verschieden sind,
d)

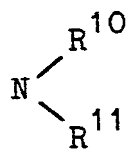

mit $R^{10}$ und $R^{11}$ gleich oder verschieden ($C_1$-$C_4$)Alkyl,
e)

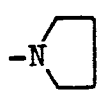
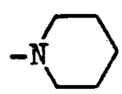
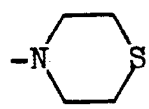
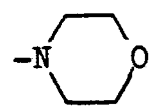

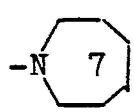
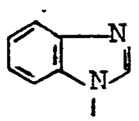
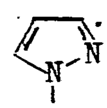
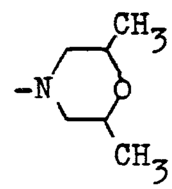

f) -OC$R^{13}$ mit $R^{13}$ gleich ($C_1$-$C_8$)-Alkyl, ($C_3$-$C_6$)-Cycloalkyl, Phenyl, Benzyl, Phenoxyphenyl, wobei die Phenylkerne jeweils unsubstituiert oder mit 1-3 F, Cl, Br, J, $CF_3$, $NO_2$, ($C_1$-$C_4$)-Alkyl oder ($C_1$-$C_4$)-Alkoxygruppen substituiert sind, die gleich oder verschieden sind,
und ihre Salze mit physiologisch verträglichen Säuren, mit Ausnahme der Verbindungen, in denen
a) gleichzeitig Z = C=O, A = Phenyl und Y = S-$CH_3$ oder S-Phenyl sind, oder
b) gleichzeitig Z = C=O,
A = Phenyl, gegebenenfalls substituiert mit 1-3 Substituenten (die unabhängig voneinander ausgewählt sind aus F,Cl,Br,J,$CF_3$, ($C_1$-$C_4$)-Alkyl und ($C_1$-$C_4$)-Alkoxy) oder 5-Chlorpyrid-2-yl und
Y = 1,2,4-Triazol-1-yl sind.

2. Verbindung nach Anspruch 1, dadurch gekennzeichnet, daß mindestens einer der Substituenten folgende Bedeutung hat:
A: Phenyl, Biphenyl, 1,2,3,4-Tetrahydronaphthyl, Thienyl, Indanyl, jeweils unsubstituiert oder im aromatischen Ring mit ein oder zwei Substituenten, die gleich oder verschieden sind und jeweils F, Cl, Br, $CF_3$, ($C_1$-$C_4$)-Alkyl oder ($C_1$-$C_4$)-Alkoxy bedeuten, substituiert sind,
Y: a)

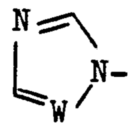

mit W = CH oder N,

b) -X-$R^8$ mit

X = O, S,

$R^8$ = ($C_1$-$C_{12}$)-Alkyl, geradkettig oder verzweigt, Phenyl, Biphenylyl, Phenoxyphenyl, Phenylthiophenyl, Phenyl-($C_1$-$C_2$)alkyl, Napthyl, Biphenylyl($C_1$-$C_2$)alkyl, Naphthyl($C_1$-$C_2$)alkyl, Benzthiazol-2-yl, Benzimidazol-2-yl, Furfuryl, Thienyl-2-methyl, Benzyl wobei die genannten Ringsysteme unsubstituiert oder durch 1, 2 oder 3 Substituenten, die gleich oder verschieden sind und jeweils F, Cl, Br, $CF_3$, Methyl, Methoxy-Gruppen bedeuten, substituiert sind,

c)

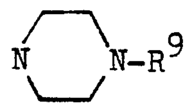

mit

$R^9$ = Phenyl, Benzyl, jeweils unsubstituiert oder im Phenylkern mit 1 oder 2 F, Cl, Br, $CF_3$, Methyl oder Methoxy-Gruppen substituiert,

Z

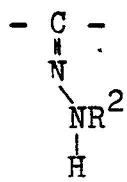 , 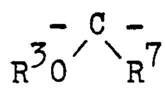 oder 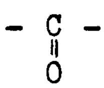

, wobei

$R^2$, $R^3$ und $R^7$ die genannten Bedeutungen haben.

3. Verbindung nach Anspruch 1, dadurch gekennzeichnet, daß mindestens einer der Substituenten folgende Bedeutung hat:

A Phenyl, Thienyl, jeweils unsubstituiert oder substituiert durch 1 oder 2 F-oder Cl-Atome, Methyl, Methoxy,

Y a)

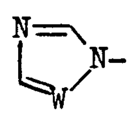

mit W = CH oder N,

b) -X-$R^8$ mit X = O, S,

$R^8$ = ($C_1$-$C_{12}$)-Alkyl, geradkettig oder verzweigt, Phenyl, Benzyl, Naphthyl, Thienylmethyl, jeweils unsubstituiert oder 1-oder 2-fach substituiert durch F, Cl, Methyl, Methoxy,

Z 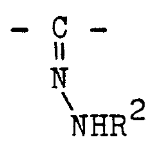 , 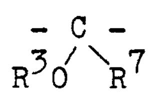 oder 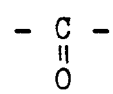 ,

wobei $R^2$, $R^3$ und $R^7$ die genannten Bedeutungen haben.

4. Verfahren zur Herstellung einer Verbindung der Formel I nach Anspruch 1 sowie ihrer Salze und Quaternisierungsprodukte, dadurch gekennzeichnet, daß man entweder

1) ein $\omega$-Chlor-halogenketon der allgemeinen Formel II

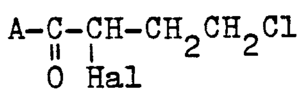 (II),

in welcher A die genannte Bedeutung und Hal für Chlor oder vorzugsweise Brom steht,

mit einem Nukleophil der Formel III

HY III,

in der Y die genannten Bedeutungen hat,

in Gegenwart eines Säureakzeptors und gegebenenfalls eines Verdünnungsmittels umsetzt oder

2) für den Fall, daß

Y= 1,2,4-Triazol-1-yl oder I midazol-1-yl ist, ein Azolylketon der Formel IV

$A-\underset{O}{\overset{\|}{C}}-\underset{Y}{C}H_2$ IV

mit 1,2-Dibromethan gegebenenfalls in Anwesenheit eines Verdünnungsmittels und eines Phasentransferkatalysators umsetzt und die erhaltenen Verbindungen der Formel I mit Z gleich CO gewünschtenfalls nach üblichen Methoden weiter umwandelt sowie gewünschtenfalls in ihre Salze oder Quaternisierungsprodukte überführt.

5. Arzneimittel enthaltend eine wirksame Menge einer Verbindung I nach Anspruch 1.

6. Verwendung einer Verbindung I nach Anspruch 1 als Arzneimittel.

7. Verwendung einer Verbindung I nach Anspruch 1 zur Herstellung eines Medikaments mit antimykotischer Wirkung.

8. Fungizid enthaltend eine wirksame Menge einer Verbindung I nach Anspruch 1.

9. Verwendung einer Verbindung I nach Anspruch 1 als Fungizid.

<u>Patentansprüche Griechenland, Spanien und Österreich:</u>

1.Verfahren zur Herstellung einer Verbindung der Formel I

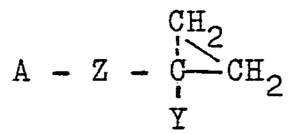

(I),

in welcher bedeuten:

A t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche

F, Cl, Br, J, $(C_1-C_8)$-Alkyl, geradkettig oder verzweigt und unsubstituiert oder mit 1-9 F-oder Cl-Atomen substituiert, $(C_3-C_8)$-Cycloalkyl, $(C_1-C_8)$-Alkoxy, geradkettig oder verzweigt, und unsubstituiert oder mit 1-9 F- oder Cl-Atomen substituiert, $(C_3-C_8)$Cycloalkyl$(C_1-C_4)$Alkyl, $(C_1-C_8)$Alkylthio, $(C_1-C_8)$-Alkylsulfinyl-und -sulfonyl, $NO_2$, CN sind;

Z C=O, $CH_2$, 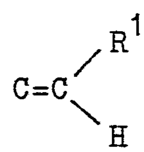 , 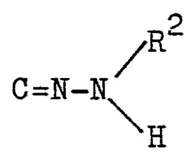 , $C=N-OR^3$ oder

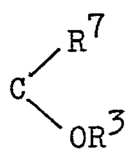

worin in diesen Gruppen Z

$R^1$ für Wasserstoff, $(C_1-C_{12})$Alkyl, Cyano, $(C_1-C_{10})$-Alkoxycarbonyl, $C_1-C_6$-Alkylcarbonyl, jeweils unsubstituiertes oder mit F, Cl, Br ein-bis dreifach substituiertes Phenylcarbonyl oder Phenyl steht, wobei die Substituenten gleich oder verschieden sind,

$R^2$ für Wasserstoff, $C_1-C_4$-Alkyl oder unsubstituiertes oder ein-bis dreifach mit F, Cl, Br substituiertes Phenyl steht, wobei die Substituenten gleich oder verschieden sind

$R^3$ Wasserstoff, $C_1-C_6$-Alkyl, $C_5-C_6$-Cycloalkyl, $(C_2-C_6)$-Alkyl, das mit 1-3 Chlor-oder Bromatomen substituiert ist, $C_2-C_6$-Alkenyl, unsubstituiert oder mit Chlor oder Brom ein-oder zweifach substituiert, $C_3-C_6$-Alkinyl, Geranyl, Farnesyl, $(C_1-C_4)$Alkoxycarbonyl-$(C_1-C_8)$-Alkyl,1,2,4-Triazolylmethyl, Phenyl$(C_1-C_4)$-alkyl oder Phenoxy$(C_1-C_6)$-alkyl, Phenyl oder Pyridyl bedeutet und die 4 letztgenannten Gruppen jeweils unsubstituiert oder ein-bis dreifach durch F, Cl, Br, $CF_3$, $(C_1-C_4)$-Alkyl-, $(C_1-C_4)$-Alkoxy-, Phenoxy-, CN,$NO_2$, COOH oder $(C_1-C_4)$-Alkoxycarbonyl substituiert sind, wobei die Substituenten gleich oder verschieden sind,

$R^3$ aber auch für Reste

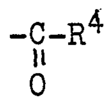 oder 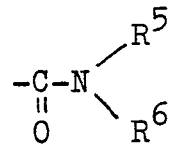

steht, in denen

$R^4$ $C_1$-$C_6$-Alkyl, $C_5$-oder $C_6$-Cycloalkyl, $C_2$-$C_6$-Alkenyl, Phenyl, Naphthyl oder Phenyl-($C_1$-$C_4$)-alkyl bedeutet, wobei die drei letztgenannten Reste unsubstituiert oder ein-bis dreifach durch F, Cl, Br, $C_1$-$C_4$-Alkyl oder $C_1$-$C_4$-Alkoxy, wobei die Substituenten gleich oder verschieden sind, oder einfach durch eine Trifluor-oder Trichlormethylgruppe substituiert sind und

$R^5$ und $R^6$ gleich oder verschieden sind und die Bedeutung von Wasserstoff, $C_1$-$C_6$-Alkyl, unsubstituiertes oder 1-3fach mit F,Cl,Br, substituiertes Phenyl haben, jedoch nicht beide gleichzeitig H oder Phenyl sein dürfen,

$R^7$ für Wasserstoff, $C_1$-$C_6$-Alkyl, $C_2$-$C_3$-Alkinyl, Phenylethinyl, Benzyl oder Phenyl steht, wobei die Phenylreste jeweils unsubstituiert oder durch 1 bis 2 F, Cl, Br, $C_1$-$C_4$-Alkyl-oder $C_1$-$C_4$-Alkoxygruppen substituiert sind, wobei die Substituenten gleich oder verschieden sind,

Y a)

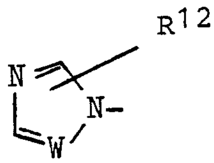

mit W = CH oder N
und $R^{12}$ gleich ($C_1$-$C_4$)-Alkyl oder $CF_3$ oder H

b) -X -$R^8$

mit X = O, S, S=O, $SO_2$ und

$R^8$= ($C_1$-$C_{12}$)-Alkyl, geradkettig oder verzweigt, ($C_3$-$C_8$)-Cycloalkyl, ($C_3$-$C_8$)Cycloalkyl-($C_1$-$C_4$)-alkyl, Phenyl, Biphenyl,Phenoxyphenylyl, Phenoxyphenyl, Phenylthiophenyl, Phenyl-($C_1$-$C_4$)-alkyl, Naphthyl, Biphenylyl -($C_1$-$C_4$)-alkyl, Phenylthiophenyl ($C_1$-$C_4$)-alkyl, Phenoxyphenyl-($C_1$-$C_4$)alkyl, Naphthyl($C_1$-$C_4$)-alkyl, Benzthiazol-2-yl, Benzimidazol-2-yl, N-($C_1$-$C_4$)-Alkylbenzimidazol-2-yl, Pyridyl, Pyrimidin-2-yl, Furfuryl, Thienyl-2-methyl, wobei die genannten Ringsysteme unsubstituiert oder durch 1, 2 oder 3 Substituenten, die gleich oder verschieden sind und jeweils F, Cl, Br, J, $CF_3$, ($C_1$-$C_4$)-Alkyl, ($C_1$-$C_4$)-Alkoxy bedeuten, substituiert sind,

c)

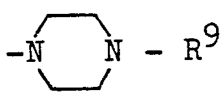

mit $R^9$= ($C_1$-$C_8$)-Alkyl, ($C_5$-$C_8$)-Cycloalkyl, Acetyl, Phenyl, Benzyl, wobei der Phenylkern jeweils unsubstituiert oder mit 1-3 F, Cl, Br, J, $CF_3$, ($C_1$-$C_4$)-Alkyl-oder ($C_1$-$C_4$)-Alkoxygruppen substituiert ist, wobei die Substituenten gleich oder verschieden sind,

d)

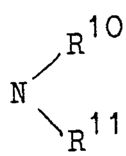

mit $R^{10}$ und $R^{11}$ gleich oder verschieden ($C_1$-$C_4$) Alkyl,

e)

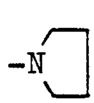 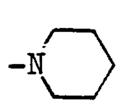 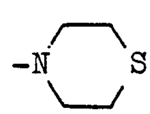 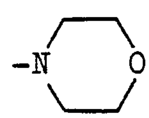

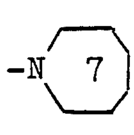 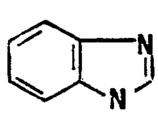 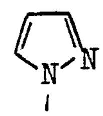 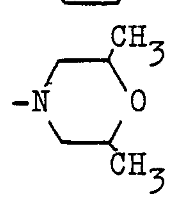

f) -$OCR^{13}$ mit $R^{13}$ gleich ($C_1$-$C_8$)-Alkyl, ($C_3$-$C_6$)-Cycloalkyl, Phenyl, Benzyl, Phenoxyphenyl, wobei die Phenylkerne jeweils unsubstituiert oder mit 1-3 F, Cl, Br, J, $CF_3$, $NO_2$, ($C_1$-$C_4$-Alkyl oder ($C_1$-$C_4$)-Alkoxygruppen substituiert sind, die gleich oder verschieden sind,

und ihre Salze mit physiologisch verträglichen Säuren, mit Ausnahme der Verbindungen, in denen

a) gleichzeitig Z = C=O, A = Phenyl und Y = S-$CH_3$ oder S-Phenyl sind, oder

b) gleichzeitig Z = C=O,

A = Phenyl, gegebenenfalls substituiert mit 1-3 Substituenten (die unabhängig voneinander ausgewählt sind aus F,Cl,Br,J,$CF_3$, ($C_1$-$C_4$)-Alkyl und ($C_1$-$C_4$)-Alkoxy) oder 5-Chlorpyrid-2-yl und

Y = 1,2,4-Triazol-1-yl sind

sowie ihrer Salze und Quaternisierungsprodukte, dadurch gekennzeichnet, daß man entweder

1) ein ω-Chlor-halogenketon der Formel II

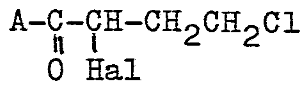

(II)

in welcher A die genannte Bedeutung und Hal für Chlor oder vorzugsweise Brom steht,

mit einem Nukleophil der Formel III

HY III,

in der Y die genannten Bedeutungen hat, in Gegenwart eines Säureakzeptors und gegebenenfalls eines Verdünnungsmittels umsetzt oder

2) für den Fall, daß

Y = 1,2,4-Triazol-1-yl oder Imidazol-1-yl ist, ein Azolylketon der Formel IV

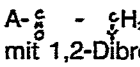 IV

mit 1,2-Dibromethan gegebenenfalls in Anwesenheit eines Verdünnungsmittels und eines Phasentransferkatalysators umsetzt und die erhaltenen Verbindungen der Formel I mit Z gleich CO gewünschtenfalls nach laborüblichen Methoden weiter umwandelt sowie gewünschtenfalls in ihre Salze oder Quaternisierungsprodukte überführt.

2. Verfahren nach Anspruch 1, dadurch gekennzeichnet, daß mindestens einer der Substituenten folgende Bedeutung hat:

A: Phenyl, Biphenylyl, 1,2,3,4-Tetrahydronaphthyl, Thienyl, Indanyl, jeweils unsubstituiert oder im aromatischen Ring mit ein oder zwei Substituenten, die gleich oder verschieden sind und jeweils F, Cl, Br, $CF_3$, ($C_1$-$C_4$)-Alkyl oder ($C_1$-$C_4$)-Alkoxy bedeuten, substituiert sind,

Y: a)

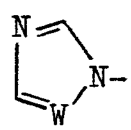

mit W = CH oder N,

b) -X-$R^8$ mit

X = O, S,

$R^8$ = ($C_1$-$C_{12}$)-Alkyl, geradkettig oder verzweigt, Phenyl, Benzyl, Biphenylyl, Phenoxyphenyl, Phenylthiophenyl, Phenyl($C_1$-$C_2$)alkyl, Naphthyl, Biphenylyl($C_1$-$C_2$)alkyl, Naphthyl($C_1$-$C_2$)alkyl, Benzthiazol-2-yl, Benzimidazol-2-yl, Furfuryl, Thienyl-2-methyl,

wobei die genannten Ringsysteme unsubstituiert oder durch 1, 2 oder 3 Substituienten, die gleich oder verschieden sind und jeweils F, Cl, Br, $CF_3$, Methyl, Methoxy-Gruppen bedeuten, substituiert sind.

c)

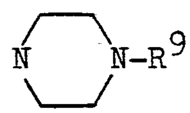

mit

$R^9$ = Phenyl, Benzyl, jeweils unsubstituiert im Phenylkern oder im Phenylkern mit 1 oder 2 F, Cl, Br, $CF_3$, Methyl oder Methoxy-Gruppen substituiert,

Z 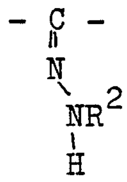 , 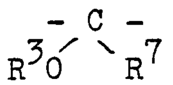 oder 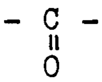

steht, wobei
$R^2$, $R^3$ und $R^7$ die genannten Bedeutungen haben.

3. Verfahren nach Anspruch 1, dadurch gekennzeichnet, daß mindestens einer der Substituenten folgende Bedeutung hat:

A Phenyl, Thienyl, jeweils unsubstituiert oder substituiert durch 1 oder 2 F-oder Cl-Atome, Methyl, Methoxy
Y a)

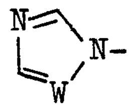

mit W = CH oder N
b) $-X-R^8$ mit X = O, S
$R^8$ = ($C_1$-$C_{12}$)-Alkyl, geradkettig oder verzweigt, Phenyl, Benzyl, Naphthyl, Thienyl-2-methyl, jeweils unsubstituiert oder 1 oder 2-fach substituiert durch F, Cl, Methyl, Methoxy,

Z 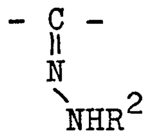 , 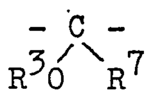 oder 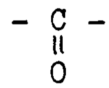 ,

wobei $R^2$, $R^3$ und $R^7$ die genannten Bedeutungen haben.

4. Verfahren zum Herstellen eines Arzneimittels, dadurch gekennzeichnet, daß man eine wirksame Menge einer nach Anspruch 1 erhaltenen Verbindung I mit pharmazeutisch üblichen Hilfsstoffen mischt.

5. Verwendung einer nach Anspruch 1 erhaltenen Verbindung I zur Herstellung eines Medikaments mit antimykotischer Wirkung.

6. Verwendung einer nach Anspruch 1 erhaltenen Verbindung I zur Herstellung eines Fungizids.